# EUROPEAN PATENT APPLICATION

(11) **EP 4 166 533 A1**
(43) Date of publication of application: **19.04.2023**
(21) Application number: 21202303.0
(22) Date of filing: 12.10.2021
(51) Int. Cl.: C07C 67/14, C07C 69/716, C07C 67/283, C07C 51/09, C07C 59/19, C07B 59/00, C07F 7/18

(54) **PROCESS FOR PRODUCTION OF KETOCARBOXYLIC ACID VINYL ESTERS**

(71) Applicant: Christian-Albrechts-Universität zu Kiel, 24118 Kiel (DE)
(72) Inventor: Herges, Rainer, 24118 Kiel (DE); Brahms, Arne, 24105 Kiel (DE); Pravdivtsev, Andrey, 24114 Kiel (DE); Hövener, Jan-Bernd, 24105 Kiel (DE)
(74) Representative: Taruttis, Stefan Georg

(57) **Abstract**

A process for producing vinyl esters of carboxylic acids, especially vinyl esters of ketocarboxylic acids, which can be α-ketocarboxylic acids or β-ketocarboxylic acids. The vinyl esters of the carboxylic acids in their vinyl group can have hydrogen and have preferably deuterium in their vinyl group. The vinyl esters can be hydrogenated with *para*-hydrogen and the spin of the para-hydrogen can be transferred to the carbonyl carbon atom of the carboxyl group, which carbonyl-carbon atom is a ¹³C, followed by hydrolysis of the ester group, producing carboxylic acids, especially ketocarboxylic acids having a hyperpolarized ¹³C in the carbonyl carbon atom of the carboxyl group.

## Description

The present invention relates to a process for producing vinyl esters of carboxylic acids, especially vinyl esters of ketocarboxylic acids, which can be α- ketocarboxylic acids or β-ketocarboxylic acids. The vinyl esters of the carboxylic acids in their vinyl group can have hydrogen and have preferably deuterium in their vinyl group. The vinyl esters can be hydrogenated with *para*-hydrogen and the spin of the *para*-hydrogen can be transferred to the carbonyl carbon atom of the carboxyl group, which carbonyl-carbon atom is a ¹³C, followed by hydrolysis of the ester group, producing carboxylic acids, especially ketocarboxylic acids having a hyperpolarized ¹³C in the carbonyl carbon atom of the carboxyl group. Transfer of the spin order created by hydrogenation of the vinyl group with para hydrogen to achieve hyperpolarization of the ¹³C carbonyl atom of the carboxyl group can be accelerated by magnetic field cycling or by suitable NMR pulse sequences.

The process for producing vinyl esters of carboxylic acids, especially vinyl esters of ketocarboxylic acids, has the advantage of a high yield, of allowing the production of deuterated vinyl esters of ¹³C-carbonyl-labelled ketocarboxylic acids, and of ketocarboxylic acids having a hyperpolarized ¹³C as the carbonyl atom.

Products of the process are 1-¹³C-hyperpolarized carboxylic acids, preferably 1-¹³C-hyperpolarized ketocarboxylic acids that are e.g. suitable for use as magnetically labelled molecules in magnetic resonance imaging (MRI) diagnostics. A preferred ketocarboxylic acid ester is pyruvic acid vinyl ester, also called vinyl pyruvate, having a hyperpolarized ¹³C in the carbonyl carbon atom of the carboxyl group for use in diagnostic ¹³C-MRI.

### State of the art

EP 3 063 119 B 1 describes producing 1-¹³C-hyperpolarized carboxylate containing compounds by providing an unsaturated ester of a carboxylate, hydrogenating the unsaturated ester with *para*-hydrogen to produce the *para*-hydrogenated ester, followed by spin order transfer to the 1-¹³C carbonyl carbon atom, e.g. by magnetic field cycling, which ester is converted by removal of the hydrogenated group to yield the 1-¹³C-hyperpolarized carboxylic acid. Particularly the allyl ester and the propargyl ester are suggested as substrates for *para*-hydrogenation because their synthesis from the corresponding alcohols and carboxylic acids is straightforward. Vinyl esters that are more efficient substrates for 1-¹³C-hyperpolarization (the spin ordered H atoms are closer to the ¹³C-carbonyl) are more difficult to prepare. A route for synthesis of vinyl pyruvate that could give acceptable yields is not described.

The unsaturated ester is kept in an organic solvent and reacted therein with *para*-hydrogen, then the magnetic field cycling is applied and the organic phase is mixed with an aqueous phase, which promotes hydrolysis of the ester and enrichment of the water-soluble 1-¹³C-hyperpolarized carboxylic acid in the aqueous phase.

Chukanov et al., ACS Omega 2018, 3, 6673-6682 describes a yield of 6% of synthesizing vinyl pyruvate from vinyl acetate and pyruvic acid with Pd^{II} acetate and KOH at 25°C for 3d.

### Object of the invention

It is an object of the invention to provide a process for efficiently producing a ketocarboxylic acid vinyl ester and its para-hydrogenation product, which after spin transfer and ester hydrolysis yields a hyperpolarized 1-¹³C carbonyl carbon compound, especially for use as a diagnostic compound in magnetic resonance imaging. Preferably, the process of the invention should produce the compound having a hyperpolarized 1-¹³C carbonyl carbon atom with high yield and at high purity. A further object is to provide a ketocarboxylic acid vinyl ester that allows for a higher efficiency of spin order transfer to the 1-¹³C carbonyl carbon.

### Description of the invention

The invention achieves the object by the features of the claims and especially provides a process in which a carboxylic acid halogenide, preferably chloride, or fluoride or bromide, of a ketocarboxylic acid is reacted with a trialkyl silylenolether for producing a ketocarboxylic acid vinyl ester and, as a by-product, a trialkyl silyl halogenide, accordingly a chloride, fluoride or bromide. The resulting ketocarboxylic acid vinyl ester can then be *para*-hydrogenated by hydrogenation with *para*-hydrogen, resulting in *para*-hydrogenation of the vinyl group. The polarisation transfer from the *para*-hydrogenated vinyl ester to the 1-¹³C carbonyl carbon of the ketocarboxylic acid can be accelerated by magnetic field cycling or by suitable NMR pulse sequences. Subsequent hydrolysis of the ester bond produces the free ketocarboxylic acid having a hyperpolarized 1-¹³C carbonyl carbon. The ketocarboxylic acid, and accordingly its carboxylic acid chloride, has a 1-¹³C carbonyl carbon. Preferably, the trialkyl silylenolether is a trialkylsilyl vinylether.

The ketocarboxylic acid can be an alpha(α)-ketocarboxylic acid or a beta(β)-carboxylic acid.

In the following reaction schemes, the halogen atom is represented by the preferred chlorine. The carboxylic acid chloride can be produced by reacting a ketocarboxylic acid to a carboxylic acid chloride according to one of the following reaction schemes:

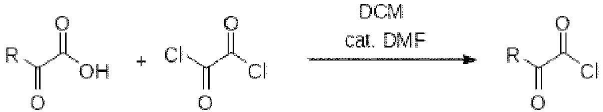

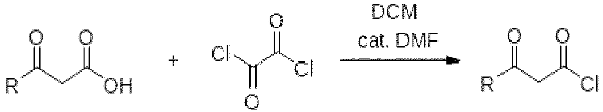

The ketocarboxylic acid chloride is produced from the corresponding ketocarboxylic acid by reacting it with a chlorinating agent, e.g. oxalyl chloride as shown here. As an alternative to the oxalyl chloride, thionyl chloride or phosphor-III-chloride or phosphor-V-chloride can be used. Preferred reagents are oxalic chloride and DMF as a catalyst, or 1-chloro-N,N,2-trimethylpropenylamine (tetramethyl-α-chlorenamine, TMCE) or any other suitable chlorinating reagent. The ketocarboxylic acid chloride in specific cases can be isolated, however, because of its instability the ketocarboxylic acid chloride is preferably further reacted in solution immediately after its production.

In a subsequent step, the α-, or β-ketocarboxylic acid halogenide, e.g. the α-, or β-ketocarboxylic acid chloride, is reacted with a trialkyl silylenolether to yield the corresponding vinylester. 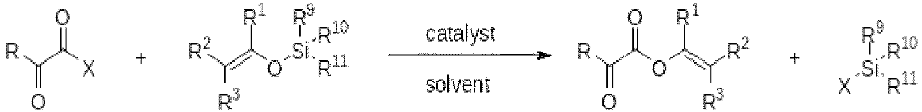 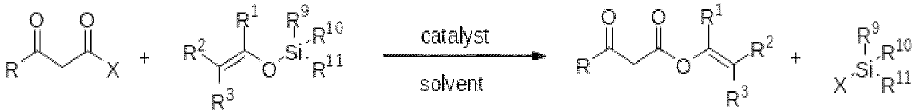 with X the halogen atom, preferably Cl, or Br or F. Upper reaction scheme for α-ketocarboxylic acid halogenide, lower reaction scheme for β-ketocarboxylic acid halogenide.

In the trialkyl silylenolether, R⁹, R¹⁰, R¹¹ can each independently be H or an alkyl, e.g. a C₁-to C₁₂- alkyl. R¹, R², R³ can each independently be H or an alkyl, e.g. a C₁- to C₁₂- alkyl, preferably each of R¹, R², R³ is D in order to produce a trialkyl silylenolether bound to a fully deuterated vinyl group.

Advantageously for hyperpolarization by subsequent *para*-hydrogenation and polarization transfer, the vinyl ester of the ketocarboxylic acid is deuterated at the vinyl group (all of R¹, R², R³ are D), and the carbonyl atom of the ketocarboxylic acid chloride is enriched in ¹³C.

In a preferred embodiment, the ketocarboxylic acid, and accordingly the acid chloride of the ketocarboxylic acid that is reacted with the trialkyl silylenolether, is fully deuterated, carrying deuterium instead of hydrogen atoms. A preferred deuterated ketocarboxylic acid is pyruvate in which the terminal methyl group is fully deuterated. Accordingly, in the preferred vinyl ester of the ketocarboxylic acid also the vinyl group is fully deuterated. A preferred vinyl ester of a ketocarboxylic acid that is obtainable by the process of the invention is of the structure 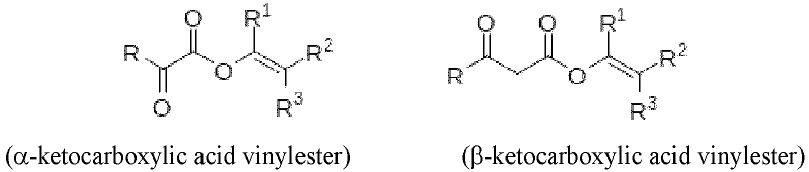 wherein R1, R2 and R3 are each deuterium, and R is a straight chain or branched C₁- to C₁₂-alkyl that preferably is fully deuterated, e.g. -CD₃, -CD₂-CD₃ , or ―(CD₂)ₙ-CD₃ with n = 1, 2, 3 or 4, or R is a straight or branched C₁- to C₁₂-alkyl with a carboxyl or carbonylester at the end that preferably is fully deuterated, e.g. -(CD₂)ₙ-COOD, -(CD₂)ₙ-COO(CD=CD₂) and wherein the carboxyl carbon of the ketocarboxylic acid is ¹³C.

In embodiments in which the vinyl group of the ketocarboxylic acid is partially deuterated, preferably fully deuterated, the spin order that is introduced into the vinyl group by *para*-hydrogenation is more effectively transferred to the ¹³C atom of the carbonyl group. Deuteration of the R group in α-ketocarboxylic acid vinylesters leads to a longer half-life (*T*₁) of the hyperpolarized ¹³C.

The catalysts suitable for the reaction of acid chlorides with trialkyl silylenolethers are transition metal salts or transition metal complexes. Among the tested catalysts, PdCl₂ is preferred as it gave the best yields.

Catalysts for the reaction of acid chlorides with trialkyl silylenolethers

| Catalyst | Eq. | Solvent | Yield^{[a]} |
|---|---|---|---|
| PdAc₂ | 0.04 | MeCN | 41 |
| PdCl₂ | 0.04 | MeCN | 80 |
| HgCl₂ | 0.04 | MeCN | 34 |

| | | | |
|---|---|---|---|
| [a] raw yield determined by ¹H-NMR after 20 h. | | | |

The systematic variation of solvents gave the following yields, showing that all solvents gave yields above 50%, with benzonitrile and dichloromethane being preferable, and with THF and acetonitrile most preferred.

| Entry | Kat. | Eq. | Solv. | Yield[%]^{[a]} |
|---|---|---|---|---|
| 1 | PdCl₂ | 0.04 | dichloromethane | 74 |
| 2 | PdCl₂ | 0.04 | THF | 80 |
| 3 | PdCl₂ | 0.04 | acetonitrile | 80 |
| 4 | PdCl₂ | 0.04 | benzonitrile | 56 |
| 5 | PdCl₂ | 0.04 | adiponitrile | 53 |
| 6 | PdCl₂ | 0.04 | toluene | 52 |
| 7 | PdCl₂ | 0.04 | 1,2 dimethoxyethane | 52 |

| | | | | |
|---|---|---|---|---|
| [a] raw yield determined by ¹H-NMR after 20 h reaction time. | | | | |

Preferably, the vinyl group of the trialkyl silylenolether is fully deuterated. Generally, a trialkyl silylenolether of a fully deuterated vinyl group can be produced by reacting fully deuterated tetrahydrofuran (THF), also termed deutero-oxolane-d₈, with butyllithium (BuLi) to give the lithium salt of the deuterated enolether of acetaldehyde, and further reacting the resulting product with a trialkylhalogensilane, e.g. trialkylchlorosilane, e.g. according to the following reaction scheme: 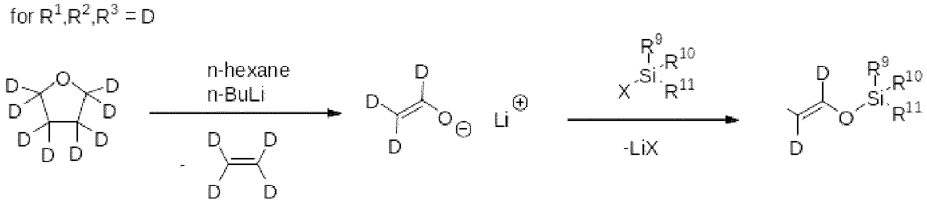 wherein X is a halogen atom, preferably chlorine.

The alkyl groups R⁹, R¹⁰, R¹¹ of the trialkylchlorosilane and accordingly of the trialkyl silylenolether of the deuterated vinyl can each be the same or can independently be selected from e.g. C₁- to C₁₂- alkyl, e.g. R9, R10 und R11 can each independently be branched alkyl, e.g. isopropyl or tert. butyl, or phenyl, e.g. R9, R10 are each methyl, R11is iso-proypl or tert.-butyl; or R9, R10 are each methyl, R11 is phenyl.

It was found that in vinyl pyruvate, with the vinyl group carrying hydrogen, after *para*-hydrogenation and applying suitable NMR pulse sequences, approx. 15% of the spin order was transferred as hyperpolarization to the 1-¹³C carbonyl carbon of the ketocarboxylic acid group, whereas the same procedure applied to a deuterated vinyl group results in a transfer of approx. 96% hyperpolarization to the 1-¹³C carbonyl carbon.

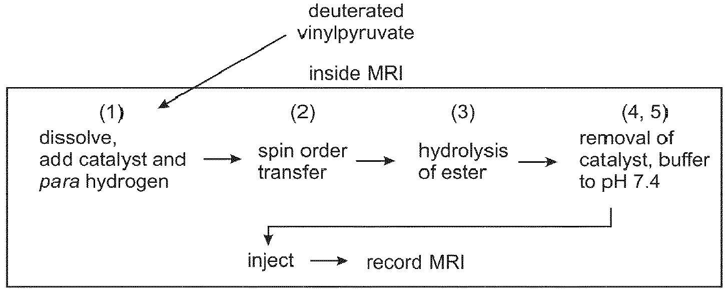

The ketocarboxylic acids either non-deuterated, fully deuterated or deuterated in their vinyl groups are stable and can be stored for extended periods of time. The following steps have to be performed in a magnetic field, preferentially inside a MRI scanner. Each step has to be performed as fast as possible because spin order and hyperpolarization decrease exponentially with relaxation.

In the process for producing a ketocarboxylic acid having a hyperpolarized carbonyl atom, the reaction mixture obtained by reacting the carboxylic acid halogenide of the ketocarboxylic acid with the trialkyl silylenolether, preferably the vinyl ester of the ketocarboxylic acid ester that is isolated from the reaction mixture, optionally with a period of storing the reaction mixture, respectively the isolated vinyl ester of the ketocarboxylic acid ester, is contacted with *para*-hydrogen for *para*-hydrogenating the vinyl group of the ketocarboxylic acid ester with subsequent spin order transfer from the *para*-hydrogen atoms to hyperpolarize a ¹³C carbonyl carbon of the ketocarboxylic acid group, and the complete reaction mixture resulting from the *para*-hydrogenation is subjected to hydrolysis of the ester bond to produce the ketocarboxylic acid having a hyperpolarized 1-¹³C carbonyl carbon, and the then resulting complete reaction mixture is subsequently subjected to separation of the ketocarboxylic acid from the reaction mixture. The separated ketocarboxylic acid is then suitable for use in diagnostics, e.g. as a contrast medium for use in MRI diagnostics.
(1) In detail, before application in vivo, the vinyl ester of the ketocarboxylic acid is dissolved in water or in an organic solvent (e.g. chloroform, pentane) and treated with *para*-hydrogen under pressure and in the presence of a suitable hydrogenation catalyst. Thereby, the intrinsic spin order of *para*-hydrogen is transferred to the vinyl group, which is now an ethyl group.
(2) Magnetic field cycling or suitable NMR pulse sequences are now applied to transfer the spin order from the ethyl group into hyperpolarization of the ¹³C atom of the neighboring carbonyl group. This hyperpolarization transfer is much more efficient if the vinyl group is deuterated. Further deuteration of the alkyl group in the ketocarboxylic acid increases the half-life (*T*₁) of the hyperpolarized state, which is in the order of only 0.5-2 minutes.
(3) Hydrolysis of the ethylester with a strong base, e.g. aqueous NaOH, yields the free hyperpolarized ketocarboxylic acid salt, e.g. pyruvate, and ethanol. In the two-phase system, the latter two compounds are in the aqueous phase and the catalyst and unreacted starting compounds are in the organic (e.g. chloroform or pentane) phase.
(4) The strongly basic aqueous solution is brought to pH 7.4 by adding a buffer. Before injecting the hyperpolarized compound in vivo, the remaining traces of the hydrogenation catalyst have to be removed because these transition metal catalysts are toxic. A simple way to remove the catalysts is filtration over an ion exchange column. Any remaining traces of organic solvent also have to be removed, e.g. by passing the solution over a column of activated charcoal. This purification step can also be applied at an earlier stage.

The purified aqueous solution of the hyperpolarized ketocarboxylic acid is now ready for injection and MRI measurement.

This embodiment has the advantage of producing the ketocarboxylic acid having a hyperpolarized 1-¹³C carbonyl carbon in a short time while providing a high yield in substance and hyperpolarization and a high purity of the ketocarboxylic acid.

For *para*-hydrogenation, a hydrogenation catalyst is added to the reaction mixture.

Preferably, the catalyst is a homogeneous hydrogenation catalyst, preferentially a rhodium complex of the general formula [Rh(diphosphine)(diene)]⁺ such as [1,4-bis-(diphenylphosphino)-butane]-(1,5-cyclooctadiene)-rhodium(I)-tetrafluoroborate (79255-71-3) or (bicyclo[2.2.1]hepta-2,5-diene)-[1,4-bis-(diphenylphosphino)-butane]-rhodium(I)-tetrafluoroborate (82499-43-2) or for hydrogenation in water, a water soluble rhodium complex with the diphosphine ligand being 1,4-bis-[(phenyl-3-propansulfonate)-phosphine]-butane disodium salt.

The process of the invention has been found to give a yield of at least 60%, e.g. up to 85%, preferably up to 95%, for α-ketocarboxylic acids. This high yield is also obtained after the separation of the ketocarboxylic acid from the reaction mixture. It is currently believed that the high yield, and accordingly a high purity of the ketocarboxylic acid is caused by the steps of the reaction, especially caused by reacting a carboxylic acid chloride of a ketocarboxylic acid with a trialkyl silylenolether, and therefore minimizing the occurrence of reverse reactions or of dimerization, e.g. Aldol-reactions as they are currently assumed to cause the very low yield in the synthesis described by Chukanov et al., ACS Omega 2018. For the same reason the yields for β-ketocarboxylic acids according to our procedure are somewhat lower than those for α-ketocarboxylic acids.

The invention is now described in greater detail by way of examples.

### Example 1: Production of trimethylsiloxyethene-D3

An oven dried 100 mL flask flushed with nitrogen was charged with 27.9 mL (24.8 g, 344 mmol) of THF-D8, (fully deuterated tetrahydrofuran). The flask was cooled to 0°C and 7.82 mL (86.0 mmol) of 11 M n-butyllithium in n-hexane was added dropwise. After 0.5 h at 0°C the solution was warmed to room temperature and after additional 0.5 h heated to 40 °C. The solution was stirred at 40 °C for 16 h and the solvent was subsequently removed in vacuo. The remaining white solid was dissolved in 20 mL dry diglyme and further residues of THF where removed in vacuo (40°C, 50 mbar). An additional amount of 30 mL dry diglyme was added and the solution was cooled to 0°C. Then 11.2 mL (88.0 mmol) of dry trimethylsilyl chloride was slowly added and a significant amount of white precipitate was formed. After stirring for 2 h at room temperature the raw product was separated from the precipitate by flash distillation of the reaction mixture at 25°C and 50 mbar (reduced to 10 mbar during the process). The raw product containing some diglyme was then further distilled at atmospheric pressure and 75°C by Kugel Rohr distillation.

### Example 2: Production of dimethylisopropylsiloxyethene-D3

An oven dried 100 mL flask flushed with nitrogen was charged with 27.9 mL (24.8 g, 344 mmol) of THF-D8. The flask was cooled to 0°C and 7.82 mL (86.0 mmol) of 11 M *n*-butyllithium in *n*-hexane was added dropwise. After 0.5 h at 0°C the solution was warmed to room temperature and after additional 0.5 h heated to 40 °C. The solution was stirred at 40 °C for 16 h and the solvent was subsequently removed in vacuo. The residue was dissolved in 50 mL of dry diethylether and cooled to 0°C. Then 10.85 mL (9.47 g, 69.3 mmol) of dry dimethylisopropylsilyl chloride were slowly added to the solution and a significant amount of white precipitate was formed. After stirring for 2 h at room temperature the solution was transferred into a separatory funnel and washed with 50 mL saturated sodium bicarbonate solution once and two times with 50 mL of distilled water. The organic layer was dried over magnesium sulfate and the solvent was removed i. vac. The obtained raw product (>95 %) was used without further purification.

### Example 3: Production of vinyl pyruvate:

In a preferred embodiment to obtain the vinyl pyruvate, an oven dried 50 mL two neck flask flushed with nitrogen was charged with 10 mL dry dichloromethane. The flask was cooled to 0°C and 0.961 mL (1.58 g, 12.5 mmol) of oxalylchloride were added. 0.787 mL (1.00 g, 11.2 mmol) of pyruvic acid were mixed with 5 mL dried dichloromethane and 13 drops of N, N-dimethylformamide. The mixture was added dropwise to the cooled solution in the two-neck flask. The reaction was stirred for 2 h at 0°C and then another 2 h at room temperature. The solution was carefully degassed under Schlenk conditions. During the process, the volume of the solution was reduced from 15 mL to 10 mL (now referred to as solution A).

In an oven-dried 50 mL two neck flask flushed with nitrogen 80.0 mg (0.450 mmol) of palladium(II)chloride were placed, and 10 mL of dry dichloromethane were added. The flask was cooled to 0°C and 1.84 mL (1.43 g, 12.34 mmol) trimethyl(vinyloxy)silane as the silylenolether were added. The solution was stirred for 0.5 h at 0°C, then the solution A was added dropwise over a time of 0.5 h at 0°C. After complete addition, the solution was allowed to warm up to room temperature and was further stirred for 20 h. The solution was transferred into a one-neck flask, a spatula tip of hydroquinone (~30 mg, 0,27 mmol) was added and the solvent was removed at reduced pressure (600 mbar 30°C). The remains were collected in 3 mL dichloromethane: *n*-pentane (1:1) and then purified by flash column chromatography using a gradient of *n*-pentane, dichloromethane. The product was isolated as a slightly yellow oil in 68% yield.

### Example 4: Production of 1- ¹³C vinyl pyruvat-D6

In a preferred embodiment to obtain the ¹³C vinyl pyruvate-D6, an oven dried 50 mL two neck flask flushed with nitrogen was charged with 10 mL dry dichloromethane. The flask was cooled to 0° C and 0.961 mL (1.58 g, 12.5 mmol) of oxalylchloride were added. 0.787 mL (1.00 g, 11.2 mmol) of ¹³C pyruvic acid were mixed with 5 mL dried dichloromethane and 13 drops of N,N-dimethylformamide. The mixture was added dropwise to the cooled solution in the two-neck flask. The reaction was stirred for 2 h at 0° C and then another 2 h at room temperature. The solution was carefully degassed under Schlenk conditions. During the process, the volume of the solution was reduced from 15 mL to 10 mL (now referred to as solution A).

In an oven-dried 50 mL three neck flask flushed with nitrogen 80.0 mg (0.450 mmol) of palladium(II)chloride were placed, and 10 mL of dry dichloromethane were added. The flask was cooled to 0° C and 1.84 mL (1.82 g, 12.38 mmol) dimethylisopropyl(vinyloxy)silane-D3 as was added. The solution was stirred for 0.5 h at 0°C, then the solution A was added using a dropping funnel over a time of 0.5 h at 0° C. After complete addition, the solution was allowed to warm up to room temperature and was further stirred for 20 h. The solution was transferred into a one-neck flask, a spatula tip of hydroquinone (~30 mg, 0,27 mmol) was added and the solvent was removed at reduced pressure (600 mbar 30°C). The remains were collected in 3 mL dichloromethane: *n*-pentane (1:1) and then purified by flash column chromatography using a gradient of *n*-pentane, dichloromethane. The product was isolated as a slightly yellow oil in 29% yield.

### Example 5: Production of divinyl oxaloacetate (oxaloacetic acid divinyl ester))

To obtain the divinyl-oxaloacetate, α-oxaloacetic acid (1.00 g, 7.57 mmol) was placed in a three necked round bottom flask under N₂ atmosphere and brought into suspension with dichloromethane (75 ml). The flask was cooled to 0 °C and N,N-dimethylformamide (13 drops) was added. Afterwards oxalyl chloride (1.17 ml, 13.7 mmol) was added dropwise as a solution in dichloromethane (10 ml). The solution was stirred for 2 h at 0 °C and another 2 h at rt. The solution was degassed under Schlenk conditions and it's volume reduced to 35 ml to obtain a solution of α-oxaloacetic acid chloride (now referred to as solution A). Palladium(II)chloride (48.5 mg, 0.274 mmol) was placed in a round bottom flask under N₂ atmosphere and brought into suspension with dichloromethane (10 ml). The suspension was cooled to 0 °C and trimethyl vinyloxy silane (2.48 ml. 16.7 mmol) was added. The mixture was stirred for 0.5 h and the prior prepared solution A was added dropwise over 0.5 h. With no further cooling the solution was stirred for 20 h. The product was obtained after flash column chromatography using a gradient of *n*-pentane and dichloromethane with a yield of 17%. Optionally, the vinyl group of the trimethyl(vinyloxy)silane was deuterated.

### Example 6: Production of divinyl α-ketoglutarate (2-oxoglutarate divinylester)

To obtain the divinyl-α-ketoglutarate, α-ketoglutaric acid (1.00 g, 6.84 mmol) was placed in a three necked round bottom flask under N₂ atmosphere and brought into suspension with dichloromethane (75 ml). The flask was cooled to 0 °C and N,N-dimethylformamide (13 drops) was added. Afterwards oxalyl chloride (1.17 ml, 13.7 mmol) was added drop wise as a solution in dichloromethane (10 ml). The solution was stirred for 2 h at 0 °C and another 2 h at rt. The solution was degassed under Schlenk conditions and its volume reduced to 35 ml to obtain a solution of α-ketoglutaric acid chloride (now referred to as solution A). Palladium(II)chloride (48.5 mg, 0.274 mmol) was placed in a round bottom flask under N₂ atmosphere and brought into suspension with dichloromethane (10 ml). The suspension was cooled to 0 °C and trimethyl vinyloxy silane (2.48 ml. 16.7 mmol) was added. The mixture was stirred for 0.5 h and the prior prepared solution A was added dropwise over 0.5 h. With no further cooling the solution was stirred for 20 h. The product was obtained after flash column chromatography using a gradient of *n*-pentane and dichloromethane with a yield of 18%. Optionally, the vinyl group of the trimethyl(vinyloxy)silane was deuterated.

### Example 7: Production of vinyl acetoacetate (vinyl-3-oxobutyrate)

To obtain the vinyl acetoacetate, lithium acetoacetate (1.00 g, 9.26 mmol) was placed in a three necked round bottom flask under N₂ atmosphere and brought into suspension with dichloromethane (75 ml) and oxalic acid (anhydrous, 0.83 g, 9.26 mmol) and stirred for 5 min. The flask was cooled to 0 °C and DMF (13 drops) was added. Afterwards oxalyl dichloride (0.791 ml, 9.26 mmol) was added dropwise as a solution in dichloromethane (10 ml). The Solution was stirred for 2 h at 0° C and another 2 h at room temperature. The solution was degassed under Schlenk conditions and its volume reduced to 35 ml to obtain a solution of acetoacetic acid chloride, which cannot be isolated or stored because it rapidly decomposes under ambient conditions. Palladium(II)chloride (65.1 mg, 0.370 mmol) was placed in a round bottom flask under N₂ atmosphere and brought into suspension with dichloromethane (10 ml). The suspension was cooled to 0 °C and trimethyl vinyloxy silane (1.51 ml. 10.2 mmol) was added. The mixture was stirred for 0.5 h and the prior prepared solution of acetoacetic acid chloride was added drop wise over 0.5 h. With no further cooling the solution was stirred for 20 h. The product was obtained with a yield of 15 %. Optionally, the vinyl group of the trimethyl(vinyloxy)silane was deuterated.

***Para* hydrogenation:** The substrate precursors 1-¹³C-vinyl pyruvate-d₃ (or d₆) (6.8 mg) and hydrogenation catalyst [1,4-bis-(diphenylphosphino)-butan]-(1,5-cyclooctadien)-rhodium(I)-tetrafluoroborat (26 mg [Rh], CAS 79255-71-3) were dissolved in 6 mL of chloroform-d. A NMR tube (5 mm diameter, medium wall, high pressure) was filled with 450 µL of the above solution (vinyl pyruvate and Rh catalyst) and connected to a gas line. The gas line was flushed with N₂ for approximately 5 s at 5 bar before connecting to the NMR tube to prevent premature hydrogenation. The NMR tube was inserted into a NMR spectrometer (WB NMR 400 MHz, Avance Neo, 9.4 T, Bruker) with the probe set to 330 K. After 2-3 minutes waiting time to reach temperature stabilization, the pressure was built up by flushing *para* hydrogen through the sample for 10-15 seconds at 7 bar. Then the *para* hydrogen bubbling was stopped and a spin order transfer sequence was executed after 2 seconds.
The same procedure can be performed with other solvents including alkanes (e.g. pentane, butane, hexane etc), acetone, and alcohols and chlorine containing solvents.

**Spin order transfer:** The spin order transfer sequences: phINEPT+, Kadlecek, Goldman (10.1016/j.jmr.2012.08.016), SEPP-INEPT (10.1016/j.pnmrs.2012.03.001), ESOTHERIC (https://doi.org/10.1002/open.201800086) and their derivatives including adiabatic and shape RF pulses (10.1021/jz501754j) or variation of magnetic field (10.1021/acs.jpcb.5b06222) were used to transfer polarization from *para*-hydrogen to ¹³C. Experimentally we used ESOTHERIC SOT sequence that provides 100% polarization of ¹³C of 1-¹³C-ethyl pyruvate-d₆ after hydrogenation. Other ketocarboxylic acid esters provide similar degrees of hyperpolarization with the same procedure.

**Ester Hydrolysis:** To perform hydrolysis, the ¹³C-hyperpolarized ethyl ester (450 µL) is pushed out of the *para*-hydrogen reaction chamber into a glass vial that is prefilled with an aqueous solution of NaOH (450 µL, 100 mmol/L). After pushing the hyperpolarized sample into the aqueous solution, the vial is shaken for about 3 seconds and let stand still to achieve phase separation for another 5-10 seconds. Then the top aqueous phase is collected. Traces of remaining catalyst are removed by pushing the aqueous phase through an ion exchange column and traces of solvent are removed by fast filtration through activated charcoal. If the ketocarboxylic ester is a pyruvic ester, the ester hydrolysis is performed in acetone-D6. Sodium pyruvate is scarcely soluble in acetone and is purified by precipitation, washing and re-dissolution in water.

The resulting hyperpolarized aqueous solution is filled into an NMR tube for quality analysis, or prefilled with enzymes or proteins for in vitro studies. If necessary, the pH of the solution is adjusted by addition of more acidic buffer to obtain the desired pH value (~7.4). After quality assurance (pH, purity, temperature) the hyperpolarized ¹³C- ketocarboxylic acid can be used for *in vivo* molecular MRI

## Claims

1. Process for producing a ketocarboxylic acid vinyl ester by reacting a carboxylic acid halogenide of the ketocarboxylic acid with a trialkyl silylenolether.

2. Process according to claim 1, **characterized in that** the carboxylic acid halogenide of the ketocarboxylic acid is produced by reacting a ketocarboxylic acid with a halogenating agent.

3. Process according to one of the preceding claims, **characterized in that** halogenide is a chloride, a fluoride or a bromide.

4. Process according to one of the preceding claims, **characterized in that** the carbonyl carbon of the ketocarboxylic acid is ¹³C or a carbon having the natural or an enriched proportion of ¹³C.

5. Process according to one of the preceding claims, **characterized in that** the enolether moiety of the trialkyl silylenolether is fully deuterated.

6. Process according to one of the preceding claims, **characterized in that** the enolether moiety of the trialkyl silylenolether is a vinyl group.

7. Process according to claim 4, **characterized in that** the trialkyl silylenolether has a fully deuterated vinyl group and is produced by reacting fully deuterated tetrahydrofuran with butyllithium (BuLi), and reacting the reaction product with a trialkylhalogensilane.

8. Process according to one of the preceding claims, **characterized in that** the ketocarboxylic acid is an α-ketocarboxylic acid or a β-ketocarboxylic acid.

9. Process according to one of the preceding claims, **characterized in that** the vinyl group is *para*-hydrogenated with *para*-hydrogen, followed by spin order transfer from the *para*-hydrogenated vinyl group to a ¹³C carbonyl carbon of the ketocarboxylic acid, hydrolysis of the ester bond and separation of the resulting hyperpolarized ketocarboxylic acid from the reaction mixture.

10. Process according to one of the preceding claims, **characterized in that** the reaction mixture obtained by reacting the carboxylic acid halogenide of the ketocarboxylic acid with the trialkyl silylenolether is contacted with *para*-hydrogen for *para*-hydrogenating the vinyl group of the ketocarboxylic acid ester with subsequent spin transfer from the *para*-hydrogen atoms to hyperpolarize a ¹³C carbonyl carbon of the ketocarboxylic acid group, the resulting complete reaction mixture is subjected to hydrolysis of the ester bond to produce the ketocarboxylic acid having a hyperpolarized 1-¹³C carbonyl carbon, and the then resulting complete reaction mixture is subsequently subjected to separation of the ketocarboxylic acid.

11. Process according to one of the preceding claims, **characterized in that** the ketocarboxylic acid is separated from a reaction mixture by evaporating the solvent and the trialkyl halogen silane by-product and/or one chromatographic separation step.

12. Process for producing deuterated trialkyl silylenolether, especially for use in a process according to one of the preceding claims, by reacting fully deuterated tetrahydrofuran (THF) with butyllithium (BuLi) and further reacting the resulting product with a trialkylhalogensilane, e.g. trialkylchlorosilane.

13. Vinyl ester of a ketocarboxylic acid having the structures: 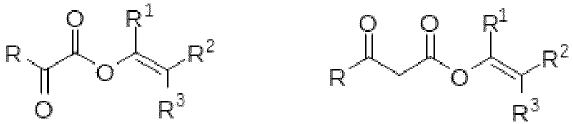 wherein R1, R2 and R3 are each deuterium, and R is a straight chain or branched C₁-to C₁₂-alkyl that is fully deuterated, and wherein the carbonyl carbon of the ketocarboxylic acid is ¹³C, and/or wherein R is a straight or branched C₁- to C₁₂-alkyl with a carboxyl or carbonylester at the end that is fully deuterated.

14. Use of a vinyl ester according to claim 13 for *para*-hydrogenation of the vinyl group with *para*-hydrogen, followed by spin order transfer from the *para*-hydrogenated vinyl group to the ¹³C carbonyl carbon of the ketocarboxylic acid, hydrolysis of the ester bond and separation of the resulting hyperpolarized ketocarboxylic acid from the reaction mixture.
